# EUROPEAN PATENT APPLICATION

(11) **EP 3 617 224 A1**
(43) Date of publication of application: **04.03.2020**
(21) Application number: 18770908.4
(22) Date of filing: 02.03.2018
(51) Int. Cl.: C07K 14/46, C12N 15/12, A61K 38/17, A61K 47/60, A61P 19/02, A61P 29/00

(54) **SELECTIVE TNFR1 ANTAGONIST PEPTIDE SN10 AND APPLICATION THEREOF IN INFLAMMATORY BOWEL DISEASE**

(30) Priority: 23.03.2017 CN 201710178897
(71) Applicant: Guilin Eight Plus One Pharmaceutical Co., Ltd., Guilin, Guangxi 541000 (CN)
(72) Inventor: LU, Yiming, Shanghai 200433 (CN); JIANG, Hailong, Shanghai 200433 (CN); BIAN, Yingying, Shanghai 200433 (CN); WANG, Jie, Shanghai 200433 (CN); LI, An, Shanghai 200433 (CN); ZHANG, Chuan, Shanghai 200433 (CN)
(74) Representative: Keil & Schaafhausen Patent- und Rechtsanwälte PartGmbB
(86) International application number: PCT/CN2018/077857
(87) International publication number: WO 2018/171405

(57) **Abstract**

The present invention relates to the field of biomedicine, and in particular to a selective TNFR1 antagonist peptide Hydrostatin-SN10 derived from the snake venom of the ringworm, having the amino acid sequence as shown in SEQ ID NO: 2. The invention also provides a selective TNFR1 antagonist peptide PEG-SN10 based on mPEG2000 modification, which is modified by covalent attachment of the carboxyl group of mPEG2000 to the free amino group of the N-terminal aspartic acid of the Hydrostatin-SN10 peptide chain. At the same time, the present invention provides Hydrostatin-SN10 and PEG-SN10 for the treatment of inflammatory bowel disease.

## Description

### Technical Field

The invention concerning to the field of biomedical technology, in particular, is a selective TNFR1 antagonistic peptide Hydrostatin-SN10 derived from the venom of *Hydrophis cyanocinctus* and its application in inflammatory bowel disease.

### Technical background

Inflammatory bowel disease (IBD) is a kind of idiopathic, chronic, inflammatory bowel diseases with a lifelong recurrence tendency, including two main types: Crohn's disease (CD) and ulcerative colitis (UC), Clinical manifestations include repeated chronic diarrhea, mucus and bloody stools, abdominal pain, abdominal mass, intestinal obstruction, perforation, body mass loss, etc., and there is a risk of malignant transformation. IBD used to be common in developed countries and is a common disease in North America and Europe. IBD used to be common in developed countries and is a common disease in North America and Europe. The incidence of UC in Europe and North America is 10/10⁵∼20/10⁵, the prevalence rate is 100/10⁵∼200/10⁵, and the incidence of CD is 5/10⁵∼10/10⁵, the prevalence rate is 50/10⁵∼100/10⁵. In recent years, the incidence of IBD has also gradually increased. Based on the statistics of several hospital cases, the prevalence rates of UC and CD are 11.6/10⁵ and 1.4/10⁵, respectively, and they are underestimated. At present, the disease has become a major cause of the digestive system and chronic diarrhea. Most of the patients are young and middle-aged, which has a great impact on social productivity and personal quality of life. It has attracted great attention from all walks of life, but there is no effective method so far. TNF-α (tumor necrosis factor) is a cytokine with various biological activities involving physiological and pathological processes such as immune regulation, inflammation, septic shock, apoptosis and autoimmunity. TNF-α is associated with a variety of inflammatory and autoimmune diseases such as rheumatoid arthritis, ulcerative colitis, asthma, and diabetes. Studies have shown that in patients with active ulcerative colitis, the expression of TNF-α is elevated, which can be seen as an inflammatory mediator to mediate the pathological damage of the colonic mucosa, and the increasing TNF-α relating to and the severity of lesions(Murch SH, Braegger CP, Walker-Smith JA, MacDonaldTT. Location of tumor necrosis factor alpha by immunohistochemistry in chronic inflammatory bowel disease[J]. Gut, 1993,34:1705-1709.).

At present monoclonal antibodies used to treat TNF-α-related diseases are effective to relieve their symptoms. However, such anti-TNF-a monoclonal antibodies completely block the biological function of TNF-α, leading to immunity to the body. The self-stabilizing and immune monitoring function bring many patients with toxic side effects that are prone to tuberculosis infection, new autoimmune diseases and even tumors. TNF-α acts on two receptors, TNFR1 and TNFR2. With the study of pathogenesis in disease, more study is currently aiming to the target of TNFR1. In general, from the perspective of anti-inflammatory, TNFR1 mainly transmits pro-inflammatory and apoptotic signals, and blocking the biological function of TNF-α by selectively blocking the signaling pathway transmitted, which has become a hot spot in the development of such kind of drugs. At present, there is no specific IBD therapeutic drug that selectively antagonizes TNFR1.

Chinese patent document CN103030687A discloses that SN1 is capable of treating diseases associated with TNF-α, and discloses that it can treat colitis induced by dextran sodium sulfate in mouse (the same as in the present example 5). However, the original SN1 (22AA) target is not specific and can bind to TNF-α, TNFR1 and TNFR2, and has the largest binding capacity to TNFR1, about 32 µM; while SN10 (10AA) is specific and selective, only Binding to TNFR1, but not to TNF-α, TNFR2; binding to TNFR1 is approximately 2.8 µM and competitive inhibition of TNFR1 binding to TNF-α. The present invention demonstrates the use of SN10 for the treatment of inflammatory bowel disease by two mouse models, colitis model induced by dextran sulfate sodium and colitis model induced by oxazolone.

### Summary of the invention

The object of the present invention is to provide a selective TNFR1 antagonist peptide Hydrostatin-SN10 derived from Qinghai snake venom and its application in inflammatory bowel disease, and another object of the present invention is to provide a selective TNFR1 based on mPEG2000 (Monomethoxypolyethylene glycol 2000) modification and use of the antagonist peptide Hydrostatin-SN10, PEG-SN10, in inflammatory bowel disease.

The inventor's research group (Jiang Hailong, 2015, Master's thesis of the Second Military Medical University of China, the structural optimization and anti-inflammatory mechanism of the anti-inflammatory active peptide Hydrodatin-SN1 of the snake venom) was intercepted by the Hydrostatin-SN1 (22AA) Shortly, Hydrostatin-SN10 (10AA) was obtained and its surface plasmon resonance technique (SPR) was used to bind to TNFR1. The binding capacity was about KD=2.8 µM, which was higher than Hydrostatin-SN1 (KD=32 µM). However, whether or not it selectively antagonizes TNFR1, it is unclear whether it can competitively inhibit the binding of TNFR1 to TNF-α; animal model results indicate that it has certain anti-inflammatory activity.

The main technical solution is: further research on Hydrostatin-SN10, surface plasmon resonance technique (SPR) and micro-thermal migration technique (MST) indicate that Hydrostatin-SN10 has specific target, can interact with TNFR1, and bind to TNFR1(about 2.8µM); And only binds to TNFR1, does not bind to TNF-α, TNFR2, competitively inhibits the interaction between TNFR1 and TNF-α, is a selective TNFR1 antagonist peptide. Hydrostatin-SN10 has significant anti-inflammatory activity in animal models(sodium dextran sulfate (DSS)-induced mouse colitis model and oxazolone (OXZ)-induced mouse colitis model). It shous that selective TNFR1 antagonist peptide Hydrostatin-SN10 can treat inflammatory bowel disease (Crohn's disease and ulcerative colitis).

Preparation of PEG-modified PEG-SN10 with the modifier mPEG2000 (monomethoxypoly), average molecular weight 2000, and struction is CH3O-(CH2CH2O)n-COOH, where n is the degree of polymerization. The carboxyl group of the mPEG2000 is covalently linked to the free amino group of the N-terminal aspartic acid to form an amide bond, thereby obtaining a PEG-SN10 modified peptide. By studying the anti-inflammatory effects of dextran sulfate sodium and oxazolone-induced acute colitis in mouse, it has proved that PEG-SN10 can inhibit the expression of inflammatory factors in mouse colon tissues and effectively ,alleviate local inflammatory symptoms and signs. IBD has a therapeutic effect.

In a first aspect of the invention provides a selective TNFR1 antagonist peptide Hydrostatin-SN10, SEQ ID No:2 shows the amino acid sequence of Hydrostatin-SN10. The Hydrostatin-SN10 is synthesized by using solid phase synthesis technology, and its purity and molecular weight are analyzed by HPLC and MS, the molecular weight is 1250.29 Dalton, and the isoelectric point is 4.39.

In a second aspect of the invention, there is provided a gene encoding of Hydrostatin-SN10, the nucleotide sequence was shown in SEQ ID NO: 1.

In a third aspect of the invention, providing the usage of Hydrostatin-SN10 for the treatment of inflammatory bowel disease.

Preferably, the medicament for treating inflammatory bowel disease is an active component which is the only TNFR1 antagonist peptide Hydrostatin-SN10, or a pharmaceutical composition comprising the selective TNFR1 antagonist peptide Hydrostatin-SN10.

According to a fourth aspect of the present invention, providing PEG-SN10 which is modified by mPEG2000 (monomethoxypolyethylene glycol 2000) based on a selective TNFR1 antagonist peptide Hydrostatin-SN10, wherein the carboxyl group of mPEG2000 is covalently linked to Hydrostatin - SN10 peptide chain N-terminal aspartic acid on the free amino group. Modification of Hydrostatin-SN10 with mPEG (monomethoxypolyethylene glycol) with an average molecular weight of approximately 2000 Daltons increased the half-life and stability of Hydrostatin-SN10. The structural formula of 4mPEG2000 can be expressed as: CH3O-(CH2CH2O)n-COOH, wherein n is a degree of polymerization, n=35-45, and the average molecular weight is 2000 Dalton.

According to a fifth aspect of the present invention, application of PEG-SN10 treating inflammatory bowel disease.

Preferably, the medicament for treating inflammatory bowel disease is: PEG-SN10 as the sole active ingredient, or a pharmaceutical composition comprising PEG-SN10. Preferably, the pharmaceutical composition and the pharmaceutically acceptable conventional pharmaceutical excipient are formulated into a pharmaceutical preparation. The pharmaceutical preparation can be a tablet, a granule, a dispersing agent, a capsule, a soft capsule, a dropping pill, an injection, a powder injection or an aerosol.

Preferably, the inflammatory bowel disease comprises Crohn's disease (CD) and ulcerative colitis (UC).

Preferably, the above-mentioned medicament for treating inflammatory bowel disease selectively antagonizes TNFR1.

The invention provides the application of the selective TNFR1 antagonist peptides Hydrostatin-SN10 and PEG-SN10 in the treatment of inflammatory bowel disease, and provides an effective inflammatory bowel disease treatment drug.

### DRAWINGS

Figure 1 The results of HPLC analysis of Hydrostatin-SN10.
Figure 2 The results of MS analysis of Hydrostatin-SN10.
Figure 3 The binding ability of Hydrostatin-SN10 to TNFR1 using BIAcore (SPR technology). Among them, A, the interaction between SN10 and TNFR1, the dissociation constant KD value is about 2.8µM; B, SN10 and TNF-α Role, no binding; C, SN10 competitive inhibition of TNF-α-TNFR1 binding (TNFR1 on the chip); D, SN10 competitive inhibition of TNF-α-TNFR1 binding (TNF-α on the chip) E, SN10 competitive inhibition of TNF-α-TNFR2 binding.
Figure 4 The binding ability of Hydrostatin-SN10 to TNFR1 by MST technique. A, The interaction between and SN10 and TNFR1, a KD value of 2.8µM. B, the interaction of SN10 and TNF-α, no binding; C, SN10 interaction with TNFR2, no binding; D, SN10 competitive inhibition of TNFR1-TNF-α binding, (TNFR1 fluorescent labeling); E, SN10 competitive inhibition of TNFR1-TNF-α binding, (TNF-α fluorescent labeling); F, SN10 competitive inhibition of TNFR2-TNF-α binding, (TNFR2 fluorescent labeling).
Figure 5 Body weight in DSS-induced acute colitis mouse model after the treatment of Hydrostatin-SN10 and PEG-SN10
Figure 6 The disease activity index in DSS-induced acute colitis mouse model after tne treatment of Hydrostatin-SN10 and PEG-SN10
Figure 7 shows the colon length in DSS-induced acute colitis mouse model after the treatment of Hydrostatin-SN10 and PEG-SN10.
Figure 8 shows the spleen index in DSS-induced acute colitis mouse model after the treatment of Hydrostatin-SN10 and PEG-SN10
Figure 9 shows the effect of myeloperoxidase activity in mouse colon tissue in DSS-induced acute colitis mouse model after the treatment of Hydrostatin-SN10 and PEG-SN10
Figure 10 is the effect of Hydrostatin-SN10 and PEG-SN10 on the expression of inflammatory cytokines in mouse serum in DSS-induced acute colitis mouse model; among them, A, TNF-α expression in mouse serum; B, IL-6 expression in mouse serum; C, IL-1β expression in mouse serum; D, IFN-γ expression in mouse serum; E, IL-10 expression in mouse serum.
Figure 11 shows the effect of Hydrostatin-SN10 and PEG-SN10 on the pathological damage of colonic tissue in DSS-induced acute colitis model, and HE staining of tissue sections (200-fold).
Figure 12 shows the effect of Hydrostatin-SN10 and PEG-SN10 on the expression of TNF-α in mouse colon tissue induced by DSS, a micrograph of tissue sections (100-fold).
Figure 13 shows body weight in OXZ-induced acute colitis mouse model after tne treatment of Hydrostatin-SN10 and PEG-SN10
Figure 14 shows the disease activity index in OXZ-induced acute colitis mouse model after tne treatment of Hydrostatin-SN10 and PEG-SN10
Figure 15 shows the colon length in OXZ-induced acute colitis mouse model after tne treatment of Hydrostatin-SN10 and PEG-SN10.
Figure 16 shows the spleen index in OXZ-induced acute colitis mouse model after tne treatment of Hydrostatin-SN10 and PEG-SN10
Figure 17 shows the effect of myeloperoxidase activity in mouse colon tissue in OXZ-induced acute colitis mouse model after tne treatment of Hydrostatin-SN10 and PEG-SN10
Figure 18 is the effect of Hydrostatin-SN10 and PEG-SN10 on the expression of inflammatory cytokines in mouse serum in OXZ-induced acute colitis mouse model; among them, A, TNF-α expression in mouse serum; B, IL-6 expression in mouse serum; C, IL-1β expression in mouse serum; D, IFN-γ expression in mouse serum; E, IL-10 expression in mouse serum.
Figure 19 shows the effect of Hydrostatin-SN10 and PEG-SN10 on the pathological damage of colonic tissue in OXZ-induced acute colitis model, and HE staining of tissue sections (200-fold).
Figure 20 shows the effect of Hydrostatin-SN10 and PEG-SN10 on the expression of TNF-α in mouse colon tissue induced by OXZ, a micrograph of tissue sections (100-fold).

### detailed description

The specific description will be described in detail examples.

The experimental methods in the following examples are conventional methods unless otherwise specified.

The experiments of Examples 2-10 were carried out using Hydrostatin-SN10 prepared in Example 1. The PEG-SN10 used in the following examples was synthesized by Qiang Yao Biotechnology Co., Ltd., and the purity was ≥98% by HPLC.

### Example 1 Synthesis and detection of a selective TNFR1 antagonist peptide Hydrostatin-SN10.

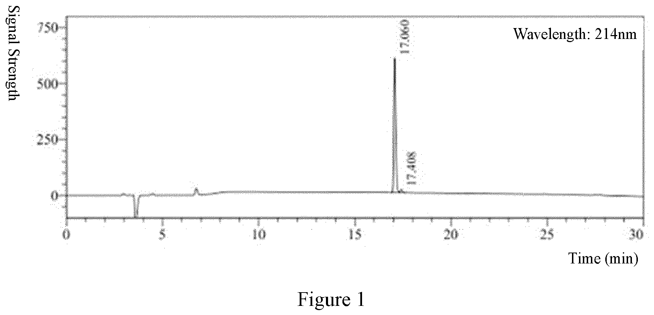

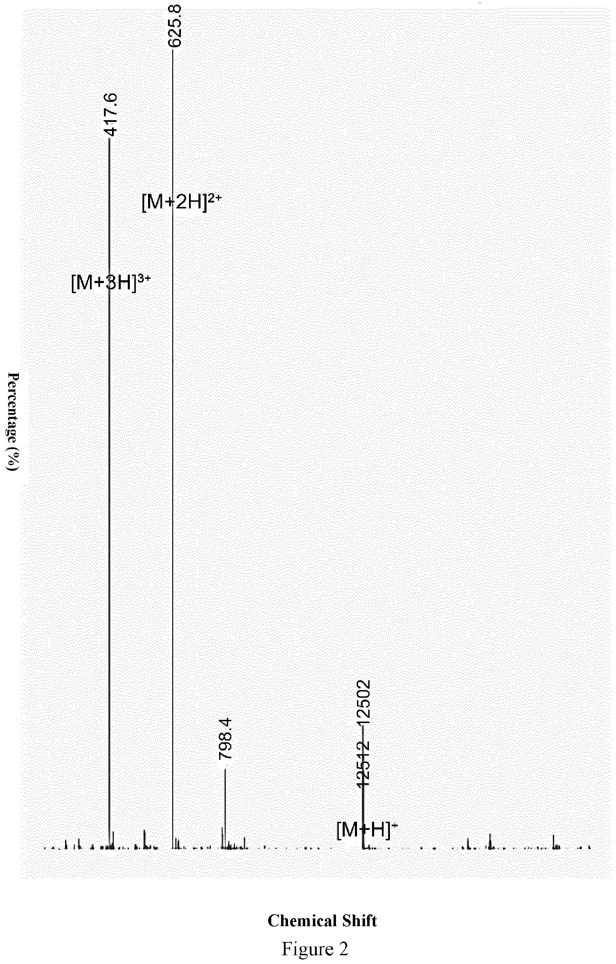

Hydrostatin-SN10 was synthesized by solid phase peptide synthesis technique, and its purity and molecular weight were analyzed by HPLC (Fig. 1) and MS (Fig. 2). The results showed that the purity was >97% and the molecular weight was 1250.29g/mol.

### Example 2 BIAcore analysis of the binding capacity of Hydrostatin-SN10 to TNFR1.

1. The running buffer flows through the channel set in the CM-5 sensor chip at a flow rate of 10µl/min until the baseline level is reached.
2. Activate the surface reactive groups of each channel of the chip with the buffer recommended by the instrument.
3. Dissolve TNFR1 and TNFR2 lyophilized powder with EP buffer, inject at a certain concentration, coat it on the surface of the chip, and then block the chip with 1 mol/L ethanolamine. The regeneration conditions are tested prior to the determination of the kinetic curve to select suitable regeneration conditions.
4. When the running buffer ran to baseline stability, a series of peptides were injected and the intermediate concentration of peptides was injected once and the response for each concentration was recorded.

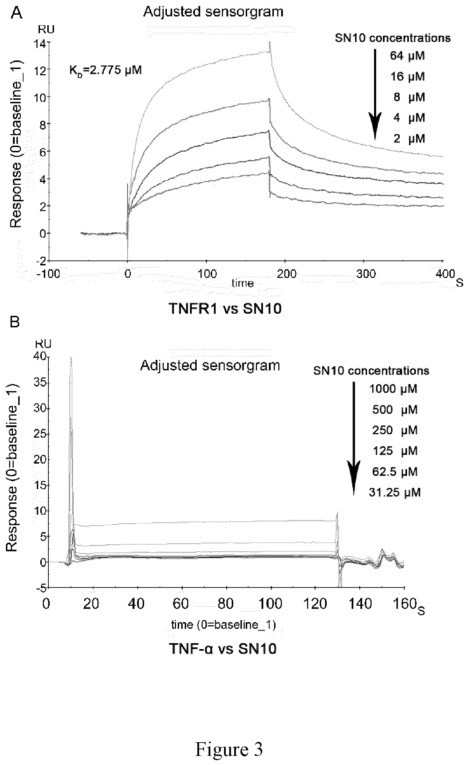

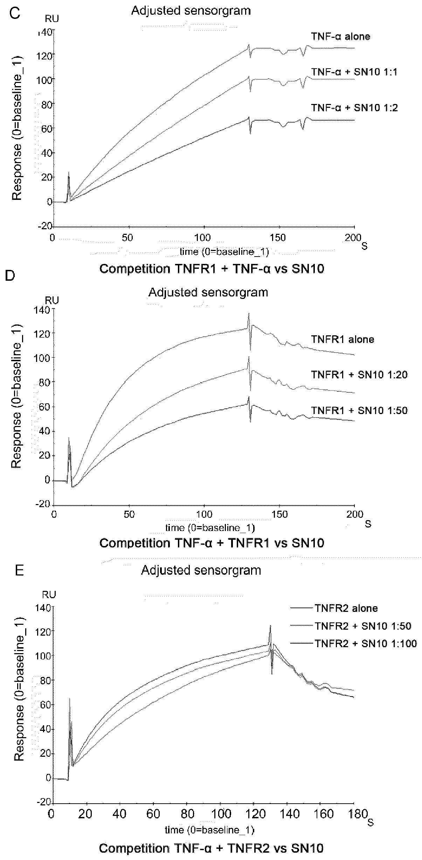

As shown in Figure 3, Hydrostatin-SN10 interacts directly with TNFR1, binding ability with TNFR1 at approximately 2.8µM; Hydrostatin-SN10 does not bind to TNF-α and competitively inhibits the interaction of TNFR1 with TNF-α.

### Example 3 MST analysis the ability of Hydrostatin-SN10 to bind to TNFR1.

1. Interaction of Hydrostatin-SN10 with TNF-α, TNFR1, TNFR2: Prepare a series of gradient concentrations of Hydrostatin-SN10 in a 1:1 dilution ratio, mix an equal volume of fluorescently labeled TNF-α/TNFR1/TNFR2 200nM with Hydrostatin-SN10, incubate in the dark for 30 min, and aspirate the appropriate amount of sample on a capillary pipette. Detect, observe the time trajectory of relative fluorescence values and the dose-response curve of thermophoresis Thermophoresis, and calculate the affinity KD value by software NTAffinityAnalysis v2.0.2 to determine whether there is a specific binding tendency.
2. Competitive inhibition of TNF-α binding to TNFR1/TNFR2 by Hydrostatin-SN10: Prepare a series of gradient concentrations of TNF-α in a 1:1 dilution ratio, mix an equal volume of fluorescently labeled TNFR1/TNFR2 200nM with TNF-α, incubate in the dark for 30 min, and aspirate the appropriate amount of sample on a capillary pipette. The KD values of the positive control TNFR1/TNFR2 and TNF-α were determined; 400nM TNFR1 and 400µM Hydrostatin-SN10 were mixed in equal volume, and then incubated with a series of concentrations of TNF-α in an equal volume for 30 min, and the appropriate amount was taken up with a capillary pipette. On-machine detection, software fitting to find the KD value. The changes of TNF-α saturation concentration, response amplitude and KD value before and after Hydrostatin-SN10 were compared.
3. Compostatin-SN10 competitive inhibition of TNF-α binding to TNFR1/TNFR2 (the same as method 2).

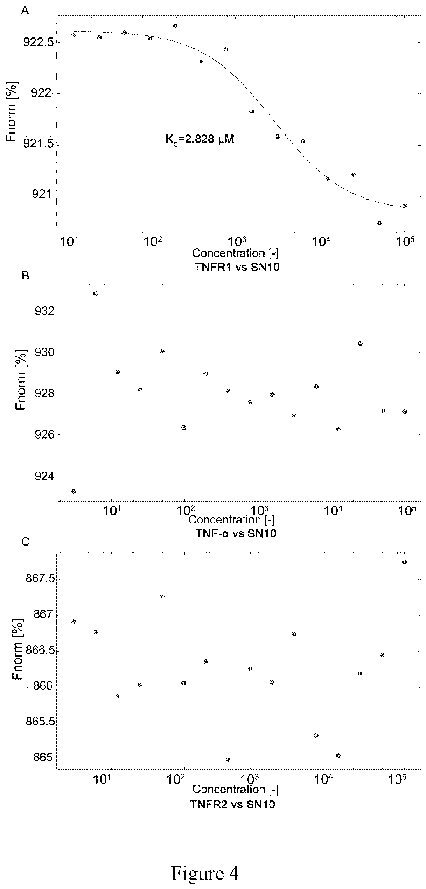

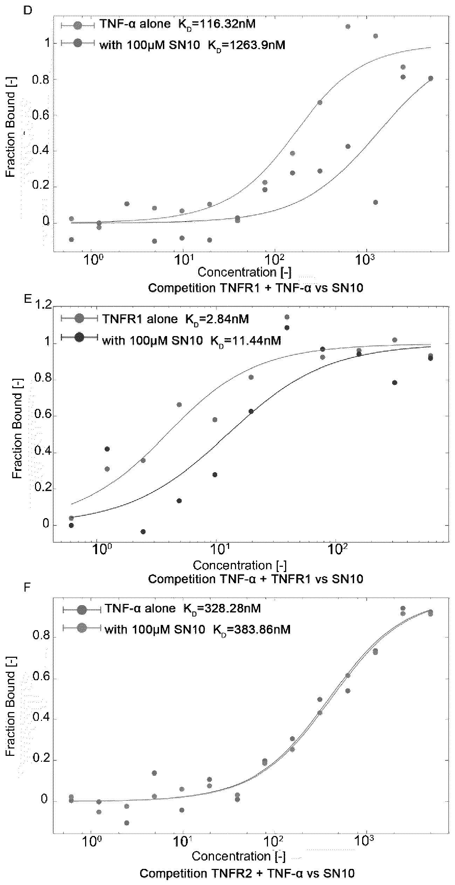

As shown in Figure 4, MST results showed that Hydrostatin-SN10 has specific target and can directly interact with TNFR1 in a binding capacity of 2.8µM ; it binds only to TNFR1 and has selectivity, but not binding with TNF-α and TNFR2 ,competitively inhibit the interaction of TNFR1 and TNF-α.

### Example 4 Detection of plasma half-life of Hydrostatin-SN10 and PEG-SN10 in SD rats.

The assay was performed according to the manufacturer's instructions using an ELISA kit purchased from Genzyme. Serum samples were collected from the posterior iliac crest with heparinized 50µL capillaries, and blood samples were collected at 1 min, 2 min, 3 min, 5 min, 10 min, 15 min, 20 min, 30 min, 45 min, 1 h, 2 h, 4 h, 6 h, 8 h after treatment. After standing for 2 h, the sample was centrifuged, and the obtained supernatant was stored at -20 ° C for testing.

**Table 1**

| Treatment | Plasma half-life(h) |
|---|---|
| Hydrostatin-SN10 | 2.18 |
| PEG-SN10 | 3.79 |

As shown in Table 1, the results show that PEG-SN 10 has a longer plasma half-life than Hydrostatin-SN10 after PEG modification.

### Example 5 Effect of Hydrostatin-SN10 and PEG-SN10 on dextran sulfate sodium-induced acute colitis mouse.

The specific implementation steps are as follows: Balb/c mice 6-8 weeks old were randomly divided into 8 groups, each group containing 10 mice.

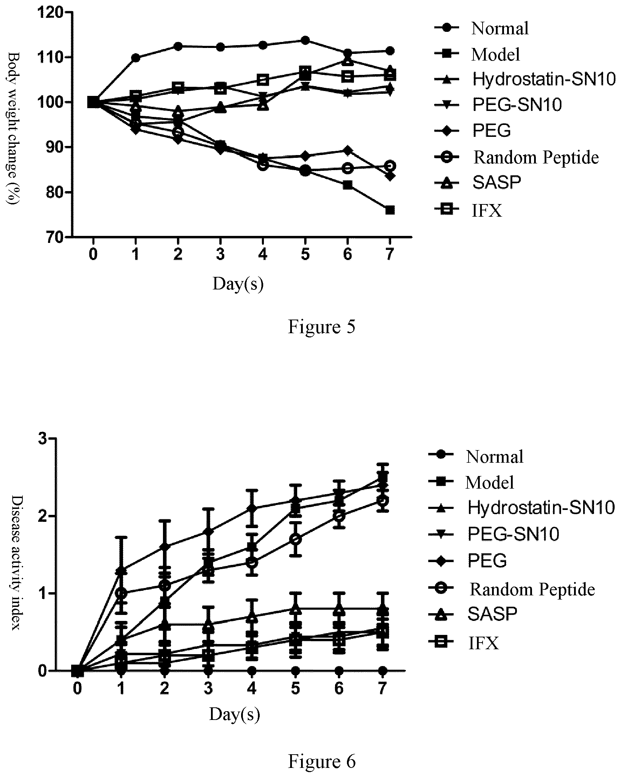

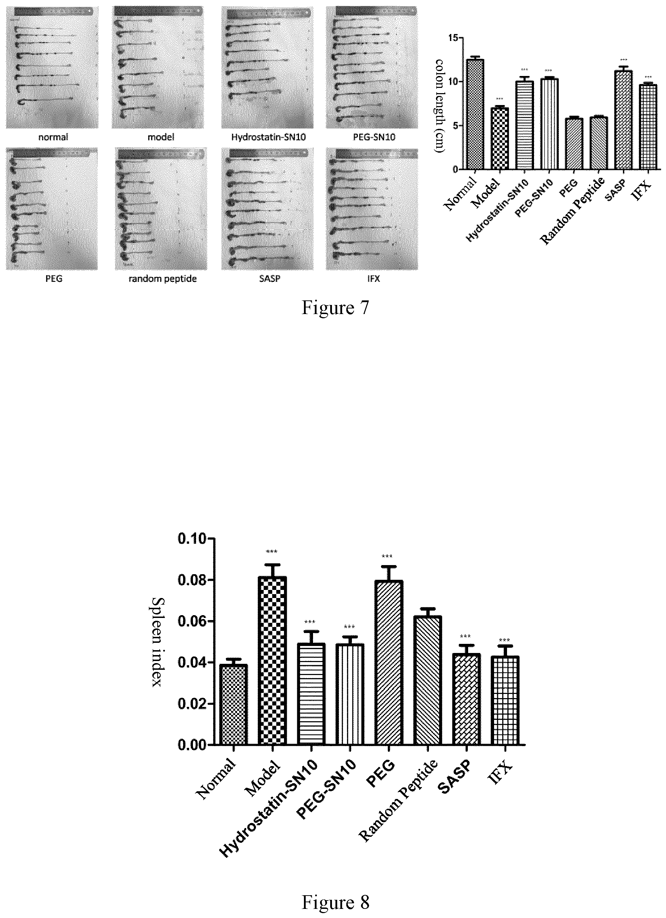

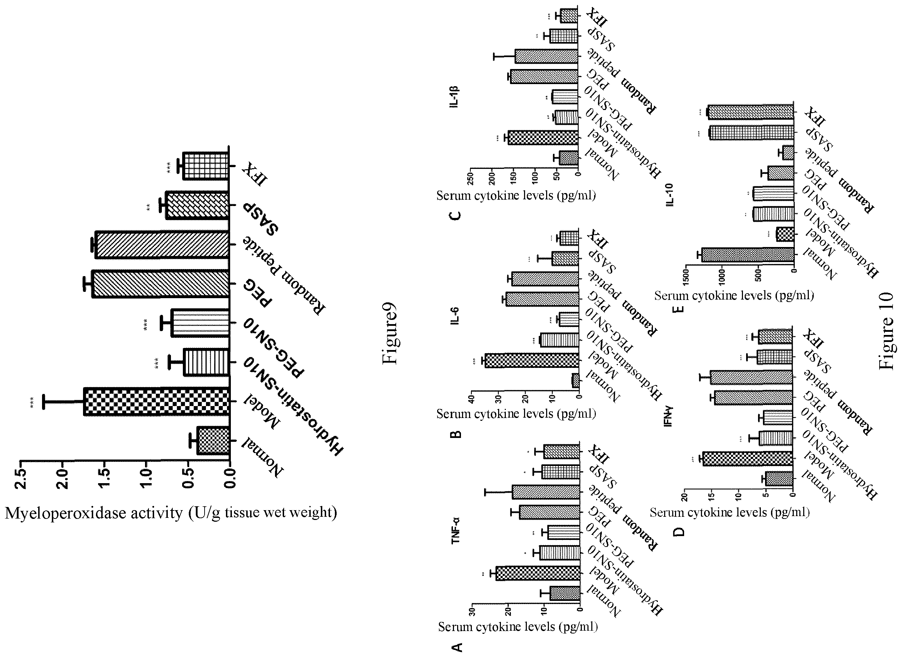

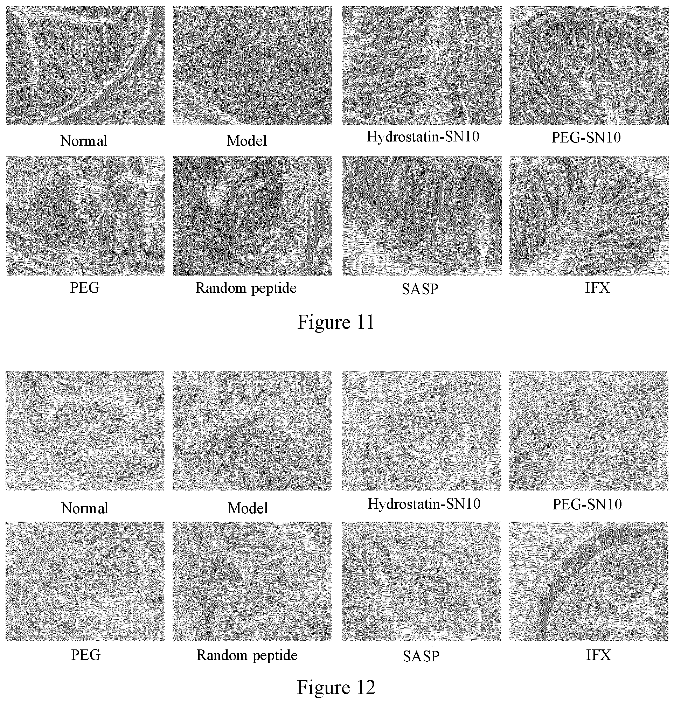

The normal group did not do any treatment; the model group mouse were given a 2.5% (w/v) DSS (commercially available from MP, MW 36,000-50,000) for 7 days in the drinking water to induce the model; other groups were intraperitoneally injected with Hydrostatin-SN10 peptide and PEG-SN10 peptide (400µg/kg/d); negative control group: random peptide group (400µg/kg/d)); positive control group Sulfasalazine (SASP, 400 mg/kg/d) and infliximab (IFX, 5 mg/kg/d). The changes in body weight of mouse in each group were recorded daily (Fig. 5), and it was found that Hydrostatin-SN10 and PEG-SN10 were effective in inhibiting the weight loss caused by colitis. According to Table 2, the disease activity index (DAI) score was obtained. As shown in Fig. 6, Hydrostatin-SN10 and PEG-SN10 can effectively alleviate the symptoms of diarrhea and blood in the stool in colitis mouse. Seven days after the model was established, the mouse were sacrificed by cervical dislocation, and the entire colon and spleen were removed. It was found that Hydrostatin-SN10 and PEG-SN10 significantly improved the colon length of the mouse (Fig. 7). The spleen weight was measured and the spleen index was calculated. It was found that Hydrostatin-SN10 and PEG-SN10 can effectively inhibit spleen changes caused by colitis (Fig. 8); Hydrostatin-SN10 was found by detecting myeloperoxidase (MPO) in mouse colon tissue. And PEG-SN10 can significantly reduce MPO activity in mouse lesional colon tissue (Fig. 9); After collecting the blood of the mice, they were allowed to rest for 2 hours, and the supernatant was taken for inflammatory factor detection. It was found that the serum levels of proinflammatory factors, TNF-α, IL-6, IL-1β, IFN-γ content were significantly decreased in the serum treated with Hydrostatin-SN10 and PEG-SN10, while the anti-inflammatory factor IL-10 was significantly increased (Fig. 10); The colonic end tissue was fixed with 10% formalin, tissue sections, and colonic changes were observed after HE staining. It was observed that Hydrostatin-SN10 and PEG-SN10 can effectively inhibit colonic lesions caused by DSS (Fig. 11); The amount of TNF-α expressed in the sections also confirmed that Hydrostatin-SN10 and PEG-SN10 can alleviate the degree of inflammation in the colon tissue of mouse (Fig. 12).

**Table 2 Scores of Disease Activity Index**

| Score | Body weight loss(%) | Stool consistency | Fecal blood |
|---|---|---|---|
| 0 | None | Normal | None |
| 1 | 1-5 | | |
| 2 | 5-10 | Loose stools | Mild |
| 3 | 10-20 | | |
| 4 | >20 | Diarrhea | Severe |

These results indicate that Hydrostatin-SN10 and PEG-SN10 are effective in treating DSS-induced colitis animal models.

### Example 6 Effect of Hydrostatin-SN10 and PEG-SN10 on oxazolone-induced acute colitis in mouse

The specific implementation steps are as follows:
Establishment of an oxazolone-induced mouse colitis animal model: Male Balb/c mice, 6-8 weeks old, were randomly divided into 8 groups, each containing 10 mice. On the first day, the back skin of the mouse was shaved (1.5 cm × 1.5 cm), and the normal group was coated with 0.15 mL of acetone/olive oil solution (acetone/olive oil volume ratio of 4:1). The model group and the SN10 group were negatively controlled. The drug and the positive control group were skin-coated with 0.15 mL of 3% (w/v) oxazolone (dissolved in acetone/olive oil solution) for skin pre-sensitization. On the 8th day, the mice were anesthetized by intraperitoneal injection of 4% chloral hydrate. The 3.5F catheter was slowly and gently inserted into the colon of the mouse about 4 cm from the anus. The normal group was injected with 0.1 mL of 50% ethanol, and the other groups were injected with 0.1 mL. 1% (w/v) oxazolone (dissolved in 50% ethanol), slowly pull out the catheter, keep the mouse vertical, head down position for 60s, so that the drug solution is fully left in the intestinal lumen. After enema, the normal group and the model group were given intraperitoneal injection of 0.1 mL of normal saline. The Hydrostatin-SN10 group, the PEG-SN10 group and the random peptide group were given 0.1 mL of Hydrostatin-SN10, PEG-SN10 and random peptide (dissolved in saline). The injection dose was 400ug/kg/d, and the positive drug group was given 0.1mL sulfasalazine and intravesical injection of infliximab (dissolved in normal saline) at doses of 400mg/kg/d and 5mg/ respectively. Each kg/d was administered continuously for 3 days.

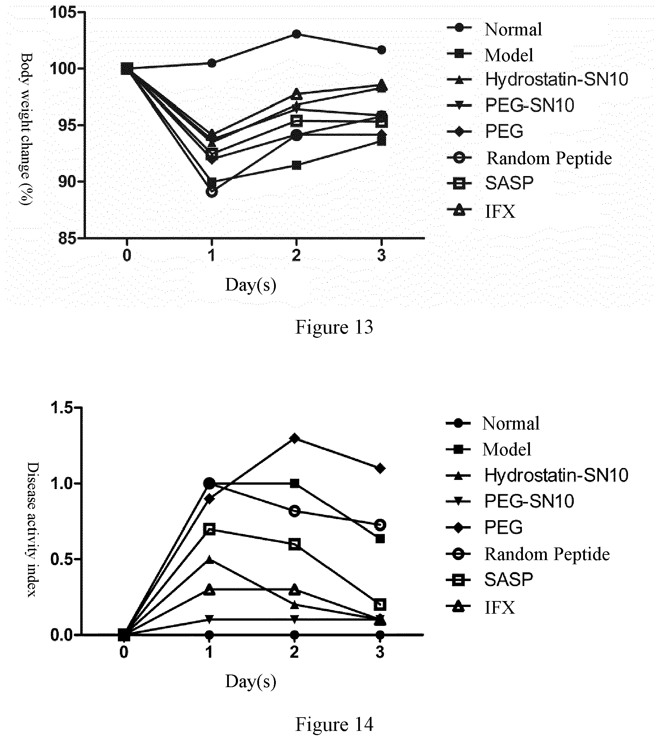

The body weight changes, stool characteristics and blood in the stool were observed and recorded daily after enema. The changes in body weight of mouse are shown in Figure 13. The results showed that Hydrostatin-SN10 and PEG-SN10 significantly reduced the weight loss. Calcuting Table 2, the disease activity index (DAI) score was shown in Fig. 14, Hydrostatin-SN10 and PEG-SN10 can effectively alleviate the symptoms of diarrhea and blood in the stool.

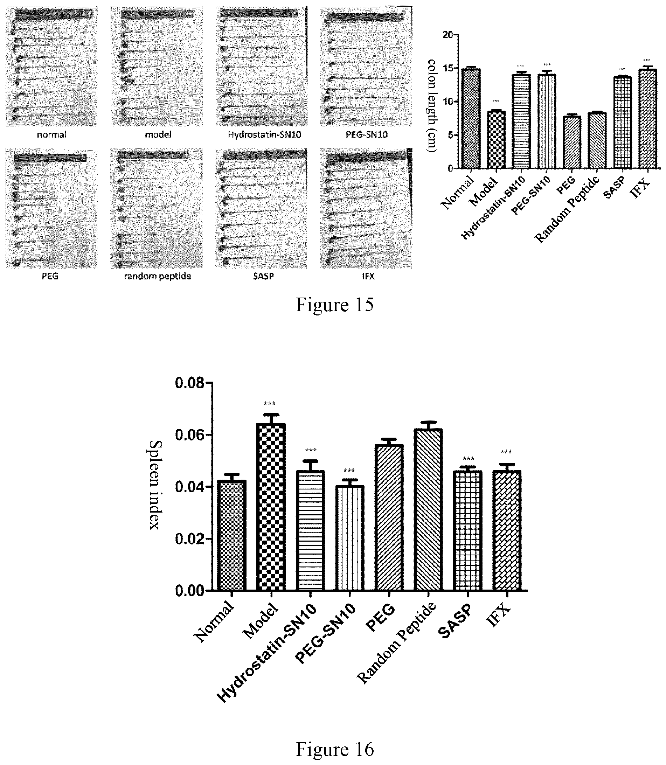

On the 3rd day after enema, the mice were sacrificed by cervical dislocation. The colon and spleen were removed immediately. The length of the colon was measured. As shown in Figure 15, Hydrostatin-SN10 and PEG-SN10 significantly improved colon length in mice. Weighing the spleen and calculating the spleen index = 10 × spleen weight (g) / body weight (g), as shown in Figure 16, Hydrostatin-SN10 and PEG-SN10 can significantly down-regulate the mouse spleen index.

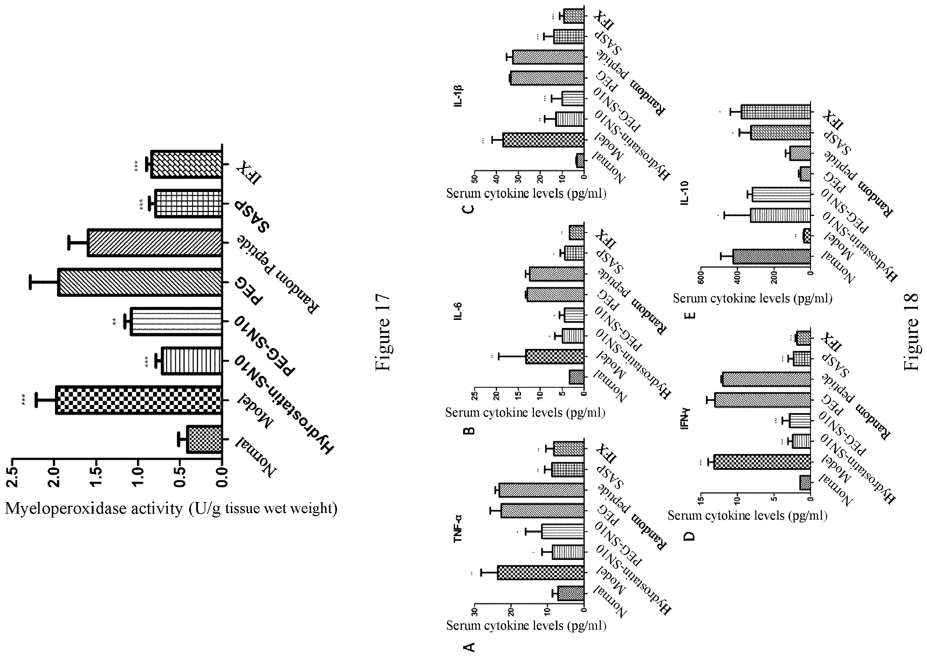

Part of the diseased colon tissue was excised and weighed. MPO activity in mouse colon tissue was detected using a myeloperoxidase (MPO) assay kit (Nanjing Institute of Bioengineering). MPO is a functional marker and activation marker for neutrophils and is involved in many processes that regulate inflammatory responses. Excessive MPO activity catalyzes the reaction to produce excess oxidants, causing local oxidative stress and oxidative tissue damage. As shown in Figure 17, Hydrostatin-SN10 and PEG-SN10 were effective in inhibiting the increase in MPO activity in inflammatory colon tissue.

As shown in Figure 18, mouse serum were taken for inflammatory factor detection, and the serum levels of proinflammatory TNF-α, IL-6, IL-1β, and IFN-γ in the serum of mice treated with Hydrostatin-SN10 and PEG-SN10 were significantly reduced, while the anti-inflammatory factor IL-10 was significantly increased.

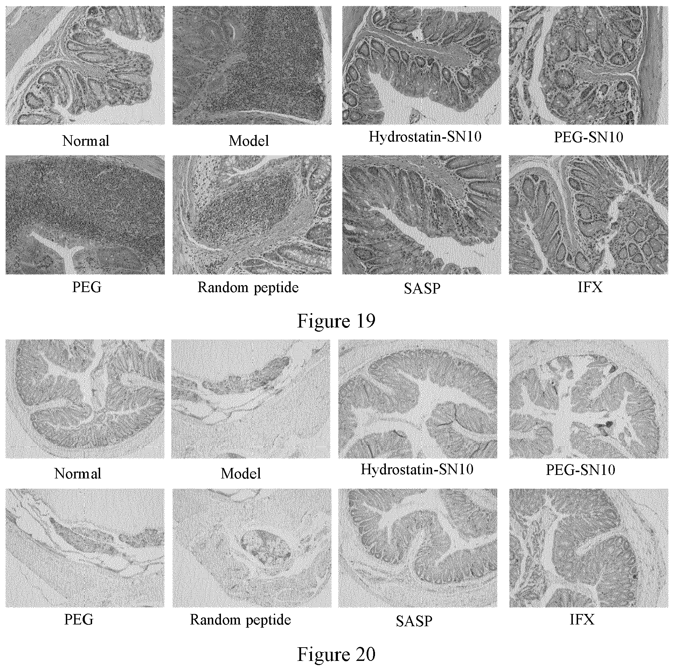

The colon of the mouse were washed with saline, fixed in 10% formalin. Tissue sections were prepared and stained with HE. As shown in Figure 19, extensive inflammatory cell infiltration was observed in the colon tissue of the model group, goblet cells were reduced, and gland density was reduced, while Hydrostatin-SN10 and PEG-SN10 significantly attenuated pathological changes in mouse colon tissue.

As shown in Figure 20, the expression of TNF-α in the colon tissue sections of the Hydrostatin-SN10 group and the PEG-SN10 group was significantly reduced. The results showed that Hydrostatin-SN10 and PEG-SN10 can alleviate the inflammation of colon tissue.

These results indicate that Hydrostatin-SN10 and PEG-SN10 are effective in the treatment of oxazolone-induced mouse colitis animal model.

The present invention have been specifically described above, but not limited to the examples, and those skilled in the art can make various changes without departing from the inventive spirit of the present invention. Modifications or substitutions, such equivalents or substitutions are intended to be included within the scope of the claims.

## Claims

1. The application of a selective TNFR1 antagonist peptide Hydrostatin-SN10 for treating induced inflammatory bowel disease, the nucleotide sequence of the gene encoding the selective TNFR1 antagonist peptide Hydrostatin-SN10 is as shown in SEQ ID NO: 1. The amino acid sequence is shown in SEQ ID NO: 2.

2. The application according to claim 1, **characterized in that** the selective TNFR1 antagonist peptide Hydrostatin-SN10 has a molecular weight of 1250.29 Daltons.

3. The application according to claim1, **characterized in that** the inflammatory bowel disease comprises Crohn's disease and ulcerative colitis; and the medicament for treating inflammatory bowel disease selectively antagonizes TNFR1.

4. The application according to claim1, **characterized in that** the medicament for treating inflammatory bowel disease is the selective TNFR1 antagonist peptide Hydrostatin-SN10 is the only active ingredient or a pharmaceutical composition comprising the selective TNFR1 antagonist peptide Hydrostatin-SN10.

5. A selective TNFR1 antagonist peptide PEG-SN10 based on mPEG2000 modification, **characterized in that** the carboxyl group of mPEG2000 is covalently linked to the free amino group of the N-terminal aspartic acid of the Hydrostatin-SN10 peptide chain, the amino acid sequence of Hydrostatin-SN10 is shown in SEQ ID NO: 2.

6. The application according to claim 5, the mPEG2000-modified selective TNFR1 antagonist peptide PEG-SN10 **characterized in that** the mPEG2000 has an average molecular weight of 2000 Daltons.

7. The application according to claim 5 or 6, mPEG2000-modified selective TNFR1 antagonist peptide PEG-SN10 used for the treatment of induced inflammatory bowel disease.

8. The application according to claim 7, **characterized in that** the drug for treating inflammatory bowel disease is a pharmaceutical composition comprising PEG-SN10 as the sole active ingredient or a pharmaceutical composition comprising PEG-SN10.

9. The application according to claim 4 or 8, pharmaceutical preparations are made up of pharmaceutical composition and the pharmaceutically acceptable conventional pharmaceutical excipient.

10. The application according to claim 9, the pharmaceutical preparation can be a tablet, a granule, a dispersing agent, a capsule, a soft capsule, a dropping pill, an injection, a powder injection or an aerosol.


SEQUENCE LISTING

<110> Guilin Bajiayi Pharmaceutical Co., Ltd.

<120> A selective TNFR1 antagonist peptide SN10 and its application in inflammatory
bowel disease

<130> /

<160> 2

<170> Patent In version 3.3

<210> 1

<211> 30

<212> DNA

<213> Artificial sequence

<400> 1

gacgaacaac acctagagac cgaactacac 30

<210> 2

<211> 10

<212> PRT

<213> Artificial sequence

<400> 2

Asp Glu Gln His Leu Glu Thr Glu Leu His

1               5                   10

SEQUENCE LISTING

<110>  Guilin Bajiayi Pharmaceutical Co., Ltd.
<120>  A selective TNFR1 antagonistic peptide SN10 and its application in
inflammatory bowel disease
<130>  /
<160>  2
<170>  PatentIn version 3.3

<210>  1
<211>  30
<212>  DNA
<213>  Artificial Sequence

<400>  1
gacgaacaac acctagagac cgaactacac                                        30


<210>  2
<211>  10
<212>  PRT
<213>  Artificial Sequence


<400>  2

Asp Glu Gln His Leu Glu Thr Glu Leu His
1               5                   10